# EUROPEAN PATENT APPLICATION

(11) **EP 2 088 418 A1**
(43) Date of publication of application: **12.08.2009**
(21) Application number: 08425070.3
(22) Date of filing: 06.02.2008
(51) Int. Cl.: G01N 1/40, A61B 10/00, B01L 3/00, G01N 1/02, G01N 33/483, G01N 33/52

(54) **A method for the integrated and automated analysis of biological samples and system for performing such a method**

(71) Applicant: Sentinel CH S.p.A., 20152 Milano (IT)
(72) Inventor: De Luca, Ugo, 20144 Milano (IT); Roveda, Luigi, 20151 Milano (IT)
(74) Representative: Cinquantini, Bruno

(57) **Abstract**

The present invention relates to an integrated and automated method for the analysis of biological samples and to a system or kit including devices adapted to perform such a method.

The method and devices for performing the method according to the present invention allow to standardise the operations of collecting the sample and simplify the operations of analysis the operator must perform, making the sample immediately available in an extremely fast and simple manner for the analysis by means of automatic laboratory instruments.

Such a method is especially advantageous for the application in the automatic analysis of biological samples which may be collected and sent to an analysis laboratory in a solid form, such as for instance in the case of faeces samples.

## Description

The present invention relates to an integrated and automated method for the analysis of biological samples and to a system or kit including devices adapted to perform such a method.

As known, it is often useful to perform laboratory analyses on samples of biological material. For instance, in the case of analyses for the qualitative and quantitative determination of one or more analytes in a biological sample, it is the patient himself/herself who must collect the sample by using different kinds of containers or supports.

The determination of the presence of analytes in a biological sample implies many steps.

The patient is responsible for the collection of the biological sample and for this purpose uses different kinds of containers, tubes, capped sterile containers of the kind used, for instance, for the collection of urine samples, and the like.

In the case of the collection of solid samples, such as for instance faeces, supports of the cardboard type may also be employed. For performing some laboratory tests, the drying of the sample on the cardboard support does not impair the success of the laboratory test. As a matter a fact, when the sample reaches the analysis laboratory, it is treated with a preset pH solution so as to obtain an aqueous solution of the components of the collected sample of biological material.

Furthermore, in many countries potentially infectious biological liquids may not be sent by mail, therefore the cardboard support allows to overcome this problem by sending a solid sample avoiding shedding in case the container is broken.

The solution is later analysed by means of spotting on a diagnostic device containing an absorbent membrane treated with specific antibodies bound to coloured compounds, which in turn bind the tested analytes giving rise to a colour signal on a preset area of the membrane.

As an alternative, the solution may be poured in sample test-tubes which may be appropriately placed on the test-tube rack of an automated analysis machine capable of performing the test for the qualitative/quantitative determination of the analyte to be detected in a substantially known manner, for instance by means of spectrophotometric analysis of the product of the reaction between the analyte and a specific reagent. To perform spectrophotometric analyses, however, the solution needs to be clear, and therefore in known processes the buffer solution is centrifuged and the supernatant is subsequently separated before performing the automated analysis, for instance by means of spectrophotometry.

In case of tests to perform on solid biological samples, semirigid supports are known to be used, such as biological sample collection devices, for instance supports made by using a paper material such as a cardboard.

In case of biological samples suitable for the collection on cardboard supports, the solid sample results being even simpler to be used by the operator performing such an analysis or in case, for instance, the sample needs to be transported or sent to an analysis laboratory far from the place where the sample has been collected.

In practice, the patient places a limited amount of the biological sample to test on such a cardboard by using, for instance, a spoon, a spatula or a stick suitable for the purpose or specifically dedicated to this use. The patient collects a limited amount of biological material and places it or spreads it on such a semirigid support.

Currently known systems provide that the collection device for the sample includes a sample carrier, consisting of a cardboard having at least one spreading area on which, as mentioned, the patient places or spreads a limited amount of the test biological sample sufficient to fill such an area. Since no special care is needed for the collection of the biological sample, the container element of the carrier may even consist of a simple cardboard which is appropriately sealed in an envelope or folded on itself.

In case, for instance, the container element of the carrier consists of a cardboard which may be folded on itself, the closing of the cardboard ensures that the material remains on the deposition area and may dry, and that the predetermined amount of material is collected on the deposition area as it is pressed between the two faces of the collection device. Therefore, when the punched cardboard strip containing the spreading area is detached, the preset amount of dry material is available for the following steps of the test.

To perform the analysis of the sample collected in this manner and dried during the transport step, in the known systems, the carrier is extracted by the operator and dipped into the preset pH solution.

The expression "buffer solution" or "preset pH solution" herein always refers to solutions featuring an optimised pH and composition depending on the analyte to be solubilized, according to the knowledge of a person skilled in the art.

The soluble components of the biological sample dissolve and the qualitative analysis of the solution may be carried out according to methods already known, i.e. spotting such a solution with the dissolved biological sample on a diagnostic device displaying a membrane sensitised with appropriate specific reagents. As an alternative, as mentioned, the quantitative automated analysis of the sample may be carried out after the buffer solution has been centrifuged in order to obtain a solution which is clear enough to be analysed by the automated instrument.

To favour the dissolving of the soluble components of the biological sample, the test tube is generally placed on a roll shaking system or on a similar system in order to wet the dry sample, for the time needed to induce the detachment from the cardboard.

These known systems force the operator to perform a sequence of manual operations which imply a considerable waste of time. Also in the case of analyses performed by means of automatic instruments, the buffer solution in which the sample has been dissolved must first be treated by centrifuging and a sequence of operations must be carried out to separate the supernatant from the solution.

Therefore, it is an object of the present invention to provide a method and a system for the automated analysis of the biological samples allowing to optimise and standardise the operations of collection and analysis of the sample, reducing the number of manual steps the operator needs to carry out.

Regarding this task, it is an object of the present invention to provide a method for the automated analysis of biological samples allowing to directly place the treated sample in standard size test-tubes adapted to be placed on the test-tube rack of an automated laboratory analyser, such as for instance an instrument for spectrophotometric analysis.

It is a further object of the present invention to provide a kit for performing such a method.

This task and these and other objects, which will become more apparent hereinafter, are achieved by a method for the analysis of biological samples, which is characterised in that it comprises the following steps: collecting a biological sample on a collection device including a semirigid carrier. Extracting said carrier from said collection device. Providing a treatment device for the biological sample including a test-tube and means adapted to separate the particulate material of the biological sample from the solution. Filling said test-tube with an appropriate amount of buffer solution having a preset pH. Placing said carrier within said test-tube by dipping it in the buffer solution. Placing and shaking the test-tube on a suitable system, for instance a roll tilting system. Actuating said means adapted to separate the particulate material from the solution thus obtaining a clear particulate-free buffer solution in at least part of said test-tube. Directly placing the treatment device containing the filtered clear buffer solution on an automatic instrument for the determination of the dissolved substances.

Further features and advantages of the present invention will become more apparent from the following detailed description, provided by way of non-limitative example and shown in the accompanying figures in which:
figure 1 shows a schematic drawing of a device for the collection of the biological sample according to the present invention in a front perspective view;
figure 2 shows a rear perspective view of the same device for the collection of the sample in figure 1;
figure 3 shows the device for the treatment of the biological sample with a buffer solution according to the present invention.

The analysis method according to the present invention provides a step of collecting the sample and a following step of treating the sample and analysing it in a laboratory.

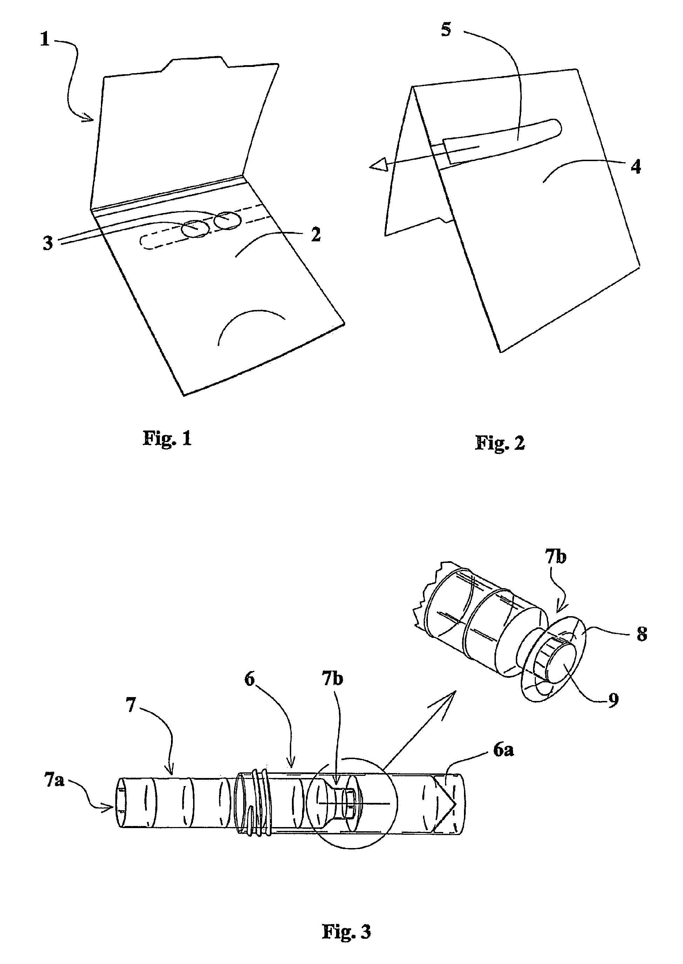

For the first step of collecting the sample, the user may employ a suitable collection device of the semirigid carrier shown for instance in figures 1 and 2.

The collection device may be configured in any way provided that it comprises a semirigid carrier, for instance a cardboard, and that it allows the collection of a predetermined amount of sample.

In the example shown in the accompanying figures 1 and 2, the collection device 1 consists of a foldable envelope that displays one or more openings 3 on an inner wall 2, the openings being shaped so as to ensure the deposition of a predetermined amount of sample. Such a device is substantially already known in the state of the art. Said openings allow the patient to directly place on the cardboard carrier 5 a limited amount of the collected biological sample for instance by means of a spoon or a stick (not shown in the figures). The carrier 5 may be obtained directly on the bottom wall 4 of the collection device 1, as shown in figure 2, or it may alternatively be introduced in a pocket which is specifically provided within the collection device.

Any configuration of the collection device 1 including a semirigid carrier 5 that may be extracted from said collection device and having the size suitable to be inserted in a laboratory test-tube is appropiate.

With the device according to figures 1 and 2, after the patient has placed the biological sample to be tested on the carrier, he/she closes the collection device 1 like a book. In this manner, the biological sample, which in the mean time will dry on the carrier, will not be accessible in any way from outside, and may be sent in any way, even by mail, to the designated analysis laboratory.

When the collection device reaches the analysis laboratory, the operator removes the cardboard carrier 5 from the device and places it in the test-tube 6 shown in figure 3.

The test-tube 6 will contain an amount of buffer solution having a preset pH and formulation together with the semirigid carrier 5, the amount being sufficient according to the known state of the art for the analyte to qualitatively and/or quantitatively be determined.

Figure 3 also shows a piston element 7 adapted to be inserted in the test-tube 6. In greater detail, the piston element 7 is a hollow cylindrical element provided with an open end 7a and ending at an opposite end 7b and in the preferred embodiment shown by way of example in figure 3, such an end is provided with a disc element 8 having an outer diameter compatible with the inner diameter of the test-tube 6. Specifically, the disc element 8 avoids the passage of material, the outer diameter adhering to the inner wall of the test-tube 6. Any other configuration of the closed end of the piston element adapted to perform the piston effect, including the configuration known from traditional syringes, may equivalently be used in place of the disc 8. The element 7 therefore behaves like the plunger of a syringe, with the difference that its closed end 7b is also provided with a porous diaphragm 9 adapted to behave as a separator filter. Air and liquid may pass through the porous diaphragm 9, while the passage of particulate material is prevented. Therefore, contrary to what occurs with the plunger of a syringe, by introducing the piston element 7 in the test-tube 6 the buffer solution contained therein passes through the porous diaphragm 9 and fills the cylindrical body of the piston element 7.

The piston element 7 provided with the porous diaphragm 9 therefore serves as a separator filter. The operator introduces the cardboard carrier 5 bearing the biological sample to be tested in the test-tube 6 containing the buffer solution. At this point, the operator must close the test-tube with its screw cap and place it on a roll shaking system, or on an equivalent system, for the purpose of wetting the dry sample, for the time required to cause it to detach from the cardboard and let the analytes contained therein dissolve, he/she must then take the cap off, take the piston element 7 and insert it in the test-tube 6 by pressing until the cardboard carrier 5 is pressed against the bottom surface 6a of the test-tube 6.

For this purpose, the bottom surface 6a of the test-tube 6 may advantageously be tapered as a cone so as to narrow towards the bottom.

As the piston 7 is pushed within the test-tube 6, the liquid-consisting buffer solution and the compounds (analytes) contained in the biological sample which have dissolved therein passes through the porous diaphragm 9 and passes within the hollow cylindrical portion of the piston element 7. In this manner, the preparation of the sample for use on automatic analysis instruments is easily obtained, without the buffer solution having to be centrifuged and poured from one container to the other in order to be analysed by an automatic analysis instrument, and more efficiently as the cardboard carrier supporting the sample is compressed against the bottom of the test-tube by the action of the piston thus performing the filtration of the solution that may immediately be used on an automatic analysis instrument.

Once the piston has been pushed against the bottom of the test-tube and the buffer solution has passed through the porous diaphragm 9 within the cylindrical body of the piston 7, the buffer solution becomes clear enough, as it is filtered by the porous diaphragm 9, to effectively be analysed by an automated analysis instrument which is especially suitable for spectrophotometric tests.

In order to automate this passage and eliminate the pouring operation by the operator, it is advantageous to provide that the treatment device, specifically the test-tube 6 has a size, specifically the outer diameter, compatible with the housings for the test-tubes in the test-tube racks of the analysis instruments. These racks usually have housings for the standard 14 or 16 mm size test-tubes. In this manner, the analysis method results being standardised and automated as the intervention of the operator is reduced at minimum.

When the laboratory receives the collection device 1 of the biological sample, the operator needs to do nothing more that extract the carrier 5 supporting the sample from the collection device, introduce it in the test-tube 6 in which the buffer solution is contained, place it on the shaker for a preset time and introduce the piston element 7 within the test-tube 6 by pressing it as if it were a syringe, thereby pressing the semirigid carrier 5 on the bottom of the test-tube.

When the buffer solution containing the elements and the test compounds of the collected biological sample dissolved in the solution is passed in the piston element 7, the test-tube 6 may be placed in the test-tube rack of an automated analysis instrument, provided to perform the spectrophotometric test for the quantitative determination of the dissolved analyte, without the operator having to pour the content or perform any test by means of other diagnostic devices.

Therefore it has been shown how the method for the analysis of biological samples disclosed up to now, as well as the use of the device system or kit disclosed herein achieves the proposed objects and purposes.

Specifically, it has been shown how the method and system according to the present invention allows to automate the analysis of a solid biological sample, the method consisting in using a semirigid carrier and a treatment device comprising a test-tube capable of very easily extracting a clear solution that may be analysed directly by the automated instruments results very effective, for instance, in the case in which an analysis to determine the presence of occult blood in faeces must be performed, as well as in the automated quantitative analysis of the faecal occult blood test (haemoglobin) in the clear solution, obtained after filtration with the test-tube/piston system, according to the present invention, by means of a specific spectrophotometric test.

Therefore by the method according to the present invention the operation of analysis of biological samples collected in a solid state may be made considerably easier, such a method results especially suitable to perform analyses on faeces samples.

It has been shown that by the method and system according to the present invention, the difficulty connected to the steps of centrifuging and preparing the solution to directly carry out the automated analysis, for instance by means of spectrophotometry, has been overcome in comparison to the known systems employing cardboard carriers in the case the analysis has to be performed by means of automated instruments.

Several modifications may be carried out by those skilled in the art without departing from the protective scope of the present invention.

Therefore, the protective scope of the claims must not be limited by the drawings or by the preferred embodiments shown by way of example in the description, but instead the claims should include all the patentable innovation features which may be deduced from the present invention, including all of the features which would be construed as equivalent by a person skilled in the art.

## Claims

1. A method for the analysis of biological samples, **characterised in that** it includes the following steps:
- collecting a biological sample on a collection device (1) including a semirigid carrier (5);
- extracting said carrier (5) from said collection device (1);
- providing a treatment device (6, 7) for the biological sample including a test-tube (6) and means (7) adapted to separate the particulate material of the biological sample from the solution;
- filling said test-tube (6) with an appropriate amount of buffer solution having a preset pH;
- placing said carrier (5) within said test-tube (6) by dipping it in the buffer solution;
- placing the test-tube to shake on a suitable system, for instance a roll tilting system;
- actuating said means (7) adapted to separate the particulate material from the solution thus obtaining a clear particulate-free buffer solution in at least one part of said test-tube;
- directly placing the treatment device (6, 7) containing the filtered clear buffer solution on an automatic instrument for the determination of the dissolved substances.

2. A method for the analysis of biological samples according to claim 1, **characterised in that** said means adapted to separate the particulate material of the biological sample from the solution include a substantially cilindrical piston element (7) provided with an open end (7a) and a closed end (7b), said closed end (7b) further including retaining means (8) adapted to perform the piston effect and at least one porous diaphragm (9).

3. A method for the analysis of biological samples according to the preceding claim, **characterised in that** the step of actuating means (7) adapted to separate the particulate material from the solution consists in compressing said carrier (5) against the bottom wall (6a) of said test-tube (6) by means of a pressure on the piston element (7) causing at the same time the passage of the buffer solution through said porous diaphragm (9) within the piston element (7), thus obtaining a clear particulate-free buffer solution within said piston element (7).

4. A method for the analysis of biological samples according to the preceding claim, **characterised in that** said retaining means adapted to perform said piston effect in said treatment device (6, 7) include a disc element (8) having a diameter compatible with the inner diameter of the test-tube (6) so that the edge of said disc effectively comes into contact with the inner surface of said test-tube (6) thus performing the piston effect.

5. A method for the analysis of biological samples according to the preceding claim, **characterised in that** said test-tube (6) has a diameter adapted to be introduced in the test-tube rack of an automated analysis instrument of the buffer solution.

6. A method for the analysis of biological samples according to any one of the preceding claims, **characterised in that** said collection device (1) consists of a foldable cardboard on one inner surface (2) of which there is provided at least one opening (3) adapted to allow the access to said carrier (5) on which said biological sample may be placed.

7. A method for the analysis of biological samples according to any one of the preceding claims, **characterised in that** said carrier (5) consists of a cardboard strip.

8. A method for the analysis of biological samples according to any one of the preceding claims, **characterised in that** said automatic instrument for the determination of dissolved compounds is an instrument capable of carrying out a spectrophotometric analysis of the clear solution.

9. A device system for the collection and analysis of biological samples, **characterised in that** it comprises a collection device (1) for biological samples including a semirigid carrier (5), and a treatment device (6, 7) for the collected biological sample, said treatment device (6, 7) comprising at least one test-tube (6) adapted to house said semirigid carrier (5) and an amount of buffer solution, and means (7) adapted to separate the particulate material of the biological sample from the solution.

10. A device system according to the preceding claim, **characterised in that** said means adapted to separate the particulate material of the biological sample from the solution include a substantially cylindrical piston element (7) provided with an open end (7a) and a closed end (7b), said closed end (7b) including retaining means (8) adapted to perform the piston effect and a porous diaphragm (9).

11. A device system according to the preceding claim, **characterised in that** said collection device (1) consists of a foldable cardboard on one inner surface (2) of which there is provided at least one opening (3) adapted to allow the access to said carrier (5) on which said biological sample may be placed.

12. A device system according to claim 11, **characterised in that** said carrier (5) consists of a cardboard strip.

13. A device system according to the preceding claim, **characterised in that** said test-tube (6) has a diameter adapted to be introduced in the test-tube rack of an automated analysis instrument of the buffer solution.

14. Use of a treatment device (6, 7) including at least one test-tube (6) and a substantially cylindrical piston element (7) provided with an open end (7a) and a closed end (7b), said closed end (7b) comprising retaining means (8) adapted to perform the piston effect and a porous diaphragm (9) to carry out the filtering through said porous diaphragm (9) of a buffer solution in the analysis process of a biological sample supported by a semirigid carrier (5) and introduced into said test-tube (6).

15. Use of a treatment device (6, 7) according to the preceding claim to directly analyse the buffer solution filtered in this manner and contained therein by an automatic analysis instrument.
